# EUROPÄISCHE PATENTSCHRIFT

(11) **EP 0 236 921 B1**
(45) Veröffentlichungstag und Bekanntmachung des Hinweises auf die Patenterteilung: **05.05.1993**
(21) Anmeldenummer: 87103035.9
(22) Anmeldetag: 04.03.1987
(51) Int. Cl.: G02B 21/00, A61B 3/00

(54) **Vorsatzeinrichtung für Mikroskope**
Supplementary device for microscopes
Dispositif adaptable pour des microscopes

(30) Priorität: 14.03.1986 DE 3608515
(43) Veröffentlichungstag der Anmeldung: 16.09.1987
(73) Patentinhaber: Oculus Optikgeräte GmbH, 35582 Wetzlar (DE)
(72) Erfinder: Spitznas, Manfred, Prof.Dr., D-5300 Bonn (DE); Veit, Wolfgang, D-6336 Solms-Burgsolms (DE); Kirchhübel, Rainer, Dipl.-Ing., D-6334 Asslar (DE)
(74) Vertreter: Missling, Arne, Dipl.-Ing.

(56) Entgegenhaltungen:
- EP-A- 0 170 857
- EP-A- 0 193 818
- DE-C- 975 226
- DE-C- 3 013 076
- DE-C- 3 437 345
- US-A- 3 656 829
- US-A- 4 231 638

## Beschreibung

Die Erfindung betrifft eine Vorsatzeinrichtung für Mikroskope, insbesondere für Stereomikroskope, für die kontaktfreie Beobachtung des durch den Patienten frei beweglichen Auges, insbesondere des Augenhintergrundes.

Für die Beobachtung des Augenhintergrundes ist es bekannt auf das Auge eine Kontaktlinse aufzusetzen, die es gestattet Bildausschnitte bis 20^{o} auf dem Fundus zu überblicken. Des weiteren ist ein sogenanntes "Panfunduskop" bekannt, das einen Fundesüberblick von bis zu 150^{o} ermöglicht. Nachteilig bei diesem bekannten Panfunduskop ist jedoch, daß dieses direkt auf das Auge aufgesetzt werden muß. Dies bedingt, daß bei Operationen, die sowohl vor der Augenlinse wie auch hinter der Augenlinse durchgeführt werden müssen, umständliche Umrüstarbeiten notwendig sind, damit beide Operationen mit einem Operations-Stereomikroskop durchgeführt werden können. Bei Verwendung nur einer Kontaktlinse ist dies zwar vermieden, jedoch ist der erhaltene Bildausschnitt mit 20^{o} doch relativ klein, so daß das Mikroskop häufig nachjustiert werden muß, was sich gleichfalls als nachteilig bei derartigen Operationen herausgestellt hat.

Ein Beispiel eins solchen Mikroskops ist in der US-A-4 231 638 beschrieben; diese Schrift offenbart zusätglich noch ein Sicherheitssystem, bei dem Augenverletzungen beim Patienten dadurch vermieden werden, daß die diesem benachbarte Linse einschließlich des zugehörigen Gehäuseteils leichtgängig axial verschiebbar ist.

Der Erfindung liegt die Aufgabe zugrunde, eine Vorsatzeinrichtung für Mikroskope, insbesondere für Stereomikroskope, tur die kontaktfreie Beobachtung der eingangs genannten Art vorzuschlagen, die einfach und schnell an das Mikroskop bzw. an das Stereomikroskop ansetzbar ist und die einen Bildausschnitt bis zu 120^{o} liefert und eine freie Beweglichkeit des Auges zuläßt und die vor allem schnell bei hohen Temperaturen sterilisiert werden kann, ohne daß die Gebrauchstüchtigkeit der Vorsatzeinrichtung beeinträchtigt wird.

Diese Aufgabe wird mit den Merkmalen des kennzeichnenden Teiles des Anspruches 1 gelöst.

Gemäß der Erfindung besteht somit die Vorsatzeinrichtung aus zwei Linsen bzw. Linsensystemen, deren Abstand zueinander einstellbar ist und die über eine Schraub- oder Bajonettkupplung mit dem Stereomikroskop verbindbar ist. Die beiden Linsen der Vorsatzeinrichtung ersetzen dann die Linse des Stereomikroskopes. Die äußere Linse, d. h. die Linse, die dem Auge des zu operierenden Patienten gegenüberliegt, ist leicht in Achsrichtung der Vorsatzeinrichtung verschiebbar, so daß auch bei einer unbeabsichtigten Bewegung des Patienten mit dem Auge, die Linse ausweicht und somit das Auge nicht verletzt werden kann. Sämtliche gegeneinander beweglichen Teile der Vorsatzeinrichtung sind mit einer Beschichtung versehen, die aus Al₂O₃ mit eingelagerten und versinterten PTFE-Polymeren besteht.

Die Vorsatzeinrichtung ist somit leicht in vorhandene Operationsmikroskope einsetzbar und ist vorteilhaft mit einem Schnellwechsel-Mechanismus versehen, mit dem wahlweise das bisherige Objektiv des Stereomikroskops oder aber die Vorsatzeinrichtung in den Strahlengang des Stereomikroskopes eingeschwenkt werden kann. Hier durch ist es möglich, einmal durch das normale Objektiv den vorderen Augenabschnitt abzubilden und beim Umschwenken auf das kontaktfreie Weitwinkelsystem, den Augenhintergrund. Optisch ist das System so aufeinander abgestimmt, daß bei diesem Hin- und Herschwenken sowohl auf dem vorderen als auch auf dem hinteren Augenabschnitt ohne weitere Verstellung ein scharfes Bild erhalten wird, sofern der Patient emmetrop ist. Bei nicht emmetropen Patienten ist das obere System zur Weitwinkel-Beobachtung verstellbar ausgebildet, so daß entsprechend nachfokusiert werden kann.

Das untere verstellbare System ist gemäß der Erfindung mit einem Gewinde versehen, in das eine Sicherheitseinrichtung eingreift, die verhindert, daß dieses vollständig herausgedreht werden kann, was zu Unfällen führen kann. Die erfindungsgemäß vorgeschlagene Beschichtung der Vorsatzeinrichtung weist eine Temperaturbeständigkeit bis 250 ^{o}C auf, so daß das komplette System bei mindestens 160 ^{o}C ohne weiteres sterilisiert werden kann. Diese Beschichtung weist darüberhinaus den Vorteil auf, daß die mechanischen Gleiteigenschaften bei der Sterilisation nicht verloren gehen, so daß ein leichten Gleiten der Teile ineinander, wie auch die Verschiebbarkeit der äußeren Linse in Achsrichtung der Vorsatzeinrichtung auch nach mehrmaligem Sterilisieren ohne Beeinträchtigung erhalten bleibt. Fette und Schmierstoffe, wie sie sonst zur Erhaltung der Leichtgängigkeit zwischen den einzelnen Teilen notwendig sind, sind hier entbehrlich.

Die äußere Linse ist vorteilhaft komplett austauschbar ausgebildet, so daß hier Linsen mit unterschiedlichen Brennweiten einsetzbar sind, wodurch verschieden große Bildausschnitte erhalten werden können.

Gemäß einer vorteilhaften Ausführungsform ist der Bildwechsler, auf den sowohl die Vorsatzeinrichtung wie auch die normale Objektivlinse des Mikroskopes angeordnet ist, mit einem Motor versehen, so daß ein schnelles Wechseln zwischen beiden optischen Systemen möglich ist.

In manchen Kliniken ist es üblich, einen sterilen Plastiksack über das komplette Mikroskop zu hängen, der normalerweise auf die Objektivfassung aufgesetzt wird. Dieser Plastiksack kann durch ein geändertes Anpaßstück auch mit der weitwinkel-Vorsatzeinrichtung verwandt werden. Zusätzlich kann auf die Vorsatzeinrichtung ein steriles Kunststoffteil von unten her aufgeschoben werden, so daß die Sterilität des Gesamtsystems erhalten bleibt.

Ein Ausführungsbeispiel der Erfindung ist im folgenden anhand der Zeichnung näher beschrieben, in dieser zeigen:
- Fig. 1: eine perspektivische Ansicht eines Stereooperationsmikroskopes mit angesetztem Adapter,
- Fig. 2: den Adapter in perspektivischer Ansicht,
- Fig. 3: eine Schnellwechseleinrichtung mit einer Vorsatzeinrichtung gemäß der Erfindung in perspektivischer Ansicht,
- Fig. 4: der untere Teil der Schnellwechseleinrichtung nach Fig. 3,
- Fig. 5: die äußere, austauschbare Linse der Vorsatzeinrichtung,
- Fig. 6: einen Schnitt nach Linie A-B in Fig. 3,
- Fig. 7: einen Schnitt durch die austauschbare äußere Linse,
- Fig. 8: ein Steuerelement für die Wechseleinrichtung nach Fig. 3,
- Fig. 9: ein Vorsatzstück für den Anschluß an den Adapter mit der normalen Objektivlinse des Mikroskopes und
- Fig. 10: in vergrößerter Darstellung einen Ausschnitt Z in Fig. 6.

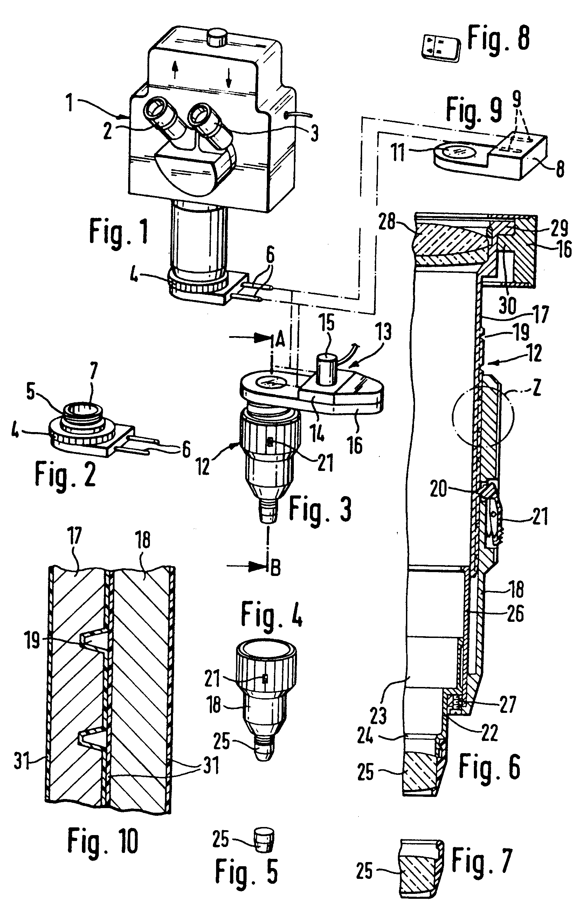

In Fig. 1 ist das Stereomikroskop insgesamt mit 1 bezeichnet. Dieses Stereomikroskop besteht aus den beiden Okularen 2, 3 mit ihren Porro-Prismen sowie den nicht weiter dargestellten Linsensystem und Vergrößerungswechsler. An das untere Ende des Mikroskops 1 ist ein Adapter 4 anstelle der üblichen Objektivlinse eingesetzt. Dieser Adapter hat, wie Fig. 2 ausweist, ein Gewindestück 5 sowie zwei Anschlußzapfen 6 und eine zentrale mittige Bohrung 7.

Sofern das Mikroskop 1 als normales Stereomikroskop benutzt werden soll, wird ein Paßstück 8 gemäß Fig. 9 an den Adapter 4 angeschoben, das zwei Bohrungen 9 aufweist, in die die Anschlußzapfen 6 des Adapters 4 eingeschoben werden. Die Objektivlinse 11 ist im Paßstück 8 eingesetzt und liegt bei angesetztem Paßstück in der optischen Achse des Mikroskops.

Die Vorsatzeinrichtung 12 kann entweder an einem gleichen Paßstück 8 befestigt sein, wie dies in Fig. 9 dargestellt ist, oder aber an einer Schnellwechseleinrichtung 13 nach Fig. 3. Die Schnellwechseleinrichtung 13 weist ein Paßstück 14 auf, das Bohrungen aufweist, in die die Anschlußzapfen 6 des Adapters 4 eingeschoben werden. An dem Paßstück 14 ist des weiteren ein Motor 15 befestigt, auf dessen Achse ein Hebel 16 drehfest befestigt ist. An den Hebel 16 ist zum einen die Vorsatzeinrichtung 12 und auf der anderen Seite im gleichen Abstand wie die Vorsatzeinrichtung 12 die Objektivlinse 11 angeordnet. Mittels dieser Schnellenwechseleinrichtung kann somit in einfacher Weise zwischen der Vorsatzeinrichtung 12 und der normalen Objektivlinse 11 gewählt werden.

In Fig. 6 ist die Vorsatzeinrichtung 12 in Seitenansicht dargestellt. Diese Vorsatzeinrichtung 12 besteht aus zwei Gehäuseteilen 17 und 18 die miteinander verschraubt sind. Hierzu ist auf der Außenseite des Gehäuseteiles 17 ein Trapezgwinde 19 ausgebildet, in das eine Nase 20 einer Sicherheitseinrichtung 21 greift. Das Trapezgewinde 19 ist so ausgebildet, daß das Gehäuseteil 18 nicht vollständig vom Gehäuseteil 19 abgedreht werden kann. Das Gehäuseteil 19 weist eine untere Öffnung 22 auf durch die ein Führungsteil 23 greift. Das Führungsteil 23 weist zum einen eine Befestigungseinrichtung 24 für eine äußere Linse 25 der Vorsatzeinrichtung 12 auf. Das Führungsteil 23 ist in einer mit dem Gehäuseteil 18 fest verbundenen Führung 26 in Achsrichtung verschiebbar angeordnet. Wird auf die äußere Linse 25 ein Druck ausgeübt, so gleitet das Führungsteil 23 in die Führung 26, so daß, falls der Patient mit seinem Auge gegen die äußere Linse 25 gestoßen ist, keine Verletzungen derselben hierdurch eintreten müssen.

Als unterer Anschlag für das Führungsteil 23 dient ein Ring 27 der mit dem Gehäuseteil 18 verschraubt ist.

Im oberen Ende des Gehäuseteiles 17 ist das innere Linsensystem 28 der Vorsatzeinrichtung 12 angeordnet. Das Ende des Gehäuses weist einen Bund 29 auf, der auf einem Ansatz 30 des Hebels 16 ruht.

Sämtliche gegeneinander verschiebbgaren Teile, wie auch das Trapezgewinde 19, sind mit einer Beschichtung 31 aus Al₂O₃ mit eingelagerten und versinterten PTFE-Polymeren überzogen, die aufgrund des Al₂O₃ eine hohe Härte aufweisen, wobei sich als Schichtmischhärte eine Härte von etwa 500 mHV ergibt. Diese Beschichtung 31 hat eine sehr gute Abriebfestigkeit und sehr gute Gleiteigenschaften, wobei der Reibungskoeffizient im trockenen Zustand bei 0,12 µ liegt. Darüberhinaus ist die Beschichtigung korrosionsbeständig gegenüber den meisten Medien, die im pH-Bereich zwischen 3 und 9 liegen. Diese Beschichtung gestattet es ohne zusätzliche Schmierstoffe auszukommen, wobei gleichfalls die gute Temperaturbeständigkeit der Schicht dafür ausschlaggebend ist, daß die Vorsatzeinrichtung bei Temperaturen über 160^{o} einfach und schnell insgesamt sterilisiert werden kann, wobei die guten Gleiteigenschaften der Beschichtung nicht verloren gehen. Diese Beschichtung macht erst den Einsatz bei einem Stereooperationsmikroskop möglich, denn bei Verwendung von Fetten und schmierstoffen käme eine Hochtemperatursterilisation nicht in Frage, da hierdurch die Fette herausgetrieben würden und über kurz oder lang die erforderliche Leichtgängigkeit nicht mehr gewährleistet wäre.

## Patentansprüche

1. Vorsatzeinrichtung für Mikroskope, insbesondere Stereomikroskope, für die kontaktfreie Beobachtung eines durch den Patienten frei beweglichen Auges, insbesondere des Augenhintergrundes, dadurch gekennzeichnet, daß die Vorsatzeinrichtung (12) zwei axial fluchtende Linsen bzw. Linsensysteme (25,28) aufweist, deren Abstand zueinander einstellbar ist und die die Objektivlinse (11) des Mikroskops (1) ersetzen, wobie die erste Linse (28), bzw. das erste Linsensystem in einem Gehäuse (17, 18) angeordnet ist; daß das Gehäuse (17,18) nach dem Entfernen der Objektivlinse (11) über eine schraub- oder bajonettartige Kupplung (4) mit dem Mikroskop (1) verbindbar ist; daß die zweite, dem Auge benachbarte Linse (25) bzw. das zweite Linsensystem in einen in einem Teil (18,) des Gehäuses (17, 18) angeordneten Führungsteil (24) angeordnet ist, das in Achsrichtung des Gehäuses (17, 18) so leicht verschiebbar angeordnet ist, daß bei einer unbeabsichtigen Bewegung des Patienten mit dem Auge die Linse (25), bzw. das Linsensystem ausweicht; und daß alle gegeneinander verschieb- und verdrehbaren Teile der Vorsatzeinrichtung (12) mit einer Beschichtung (31) versehen sind, die aus Al₂O₃ mit eingelagerten und versinterten PTFE-Polymeren besteht.

2. Vorsatzeinrichtung nach Anspruch 1, dadurch gekennzeichnet, daß die Vorsatzeinrichtung (12) einen Adapter (4) aufweist, der mit dem Mikroskop (1) verschraubt ist, daß am Adapter (4) eine Schnell-Wechseleinrichtung (13) ansetzbar ist, die eine Drehachse aufweist, an der ein Hebel (16) befestigt ist, an dem im gleichen Abstand von der Drehachse auf der einen Seite die Vorsatzeinrichtung (12) und auf der anderen Seite die normale Objektivlinse (11) des Mikroskops angeordnet ist.

3. Vorsatzeinrichtung nach Anspruch 1 oder 2, dadurch gekennzeichnet, daß die Schnell-Wechseleinrichtung (13) über Anschlußzapfen (6) an den Adapter (4) angeschlossen ist.

4. Vorsatzeinrichtung nach einem der Ansprüche 1 bis 3, dadurch gekennzeichnet, daß die Vorsatzeinrichtung (12) aus zwei miteinander verschraubten Gehäuseteilen (17,18) besteht, die mit einer Sicherungseinrichtung (21) gegen ein unbeabsichtigtes Trennen versehen sind.

5. Vorsatzeinrichtung nach Anspruch 4, dadurch gekennzeichnet, daß die Sicherungseinrichtung (21) aus einer federbelasteten Nase (20) besteht, die in dem einen Gehäuseteil (18) gelagert ist und in ein am zweiten Gehäuseteil (17) ausgebildetes Trapezgewinde (19) eingreift.

6. Vorsatzeinrichtung nach einem der Ansprüche 1 bis 5, dadurch gekennzeichnet, daß die Arbeitsstellung der äußeren Linse (25) durch einen im zweiten Gehäuseteil eingesetzten Anschlag (27) bestimmt ist.

7. Vorsatzeinrichtung nach Anspruch 6, dadurch gekennzeichnet, daß der Anschlag (27) aus einem Ring gebildet ist.

8. Vorsatzeinrichtung nach einem der Ansprüche 1 bis 6, dadurch gekennzeichnet, daß die Drehachse der Schnell-Wechseleinrichtung (13) an einen Antriebsmotor (15) gekoppelt ist.

9. Vorsatzeinrichtung nach einem der vorhergehenden Ansprüche, dadurch gekennzeichnet, daß die Sicherungseinrichtung (21) gleichzeitig die Gewindemutter bildet.

## Claims

1. An accessory for microscopes, in particular stereomicroscopes, for the contact-free observation of an eye, in particular of the back of the eye, which eye is freely movable by the patient, characterised in that the accessory (12) has two axially aligned lenses or lens systems (25,28), whose distance from one another is adjustable and which replace the objective lens (11) of the microscope (1), the first lens (28) or first lens system being arranged in a housing (17,18); in that, after the objective lens (11) has been removed, the housing (17,18) can be connected to the microscope (1) via a screw-type or bayonet-type coupling (4); in that the second lens (25) or second lens system, which is adjacent to the eye, is arranged in a guide part (24), which is arranged in a part (18) of the housing (17,18) and is easily displaceable in the axial direction of the housing (17,18) so that the lens (25) or lens system gives way when the patient unintentionally moves his eye; and in that all parts of the accessory (12) which can be displaced and rotated relative to one another are provided with a coating (31) consisting of Al₂O₃ with embedded and sintered PTFE polymers.

2. An accessory according to Claim 1, characterised in that the accessory (12) has an adapter (4) which is screwed to the microscope (1), and in that a quick-change device (13) can be attached to the adapter (4), this quick-change device (13) having a fulcrum at which a lever (16) is mounted, the accessory (12) being arranged on one side of this lever (16) and the standard objective lens (11) of the microscope being arranged on the other side of this lever (16), both at an equal distance from the fulcrum.

3. An accessory according to Claim 1 or 2, characterised in that the quick-change device (13) is connected to the adapter (4) via connecting pins (6).

4. An accessory according to any one of Claims 1 to 3, characterised in that the accessory (12) consists of two housing parts (17,18) which are screwed together and which are provided with a locking device (21) to prevent them from being separated unintentionally.

5. An accessory according to Claim 4, characterised in that the locking device (21) consists of a spring-loaded lug (20) which is mounted in one housing part (18) and engages in a trapezoidal thread (19) formed on the second housing part (17).

6. An accessory according to any one of Claims 1 to 5, characterised in that the working position of the outer lens (25) is determined by a limit stop (27) positioned in the second housing part.

7. An accessory according to Claim 6, characterised in that the limit stop (27) is formed from a ring.

8. An accessory according to any of Claims 1 to 6, characterised in that the fulcrum of the quick-change device (13) is coupled to a driving motor (15).

9. An accessory according to any one of the preceding Claims, characterised in that the locking device (21) simultaneously forms the threaded nut.

## Revendications

1. Dispositif adaptable sur des microscopes, en particulier sur des stéréomicroscopes, pour observer, sans contact, l'oeil d'un patient, en particulier le fond arrière de l'oeil, en gardant la mobilité de l'oeil, caractérisé en ce que le dispositif adaptable (12) présente deux lentilles, ou systèmes de lentilles, (25, 28), alignées axialement, dont on peut régler la distance relative et qui remplacent la lentille (11) de l'objectif du microscope (1), la première lentille (28), ou le premier système de lentilles, étant disposé dans un boîtier (17, 18); en ce que le boîtier (17, 18) peut s'adapter sur le microscope (1) au moyen d'une fixation (4) à vis ou à baïonnette, après enlèvement de la lentille (11) de l'objectif; en ce que la deuxième lentille (25), la plus proche de l'oeil, ou le deuxième système de lentilles, est disposée dans une pièce de guidage (24), disposée elle-même dans une pièce (18) du boitier (17, 18), pièce de guidage (24) qui peut facilement se déplacer suivant la direction axiale du boitier (17, 18) de sorte que, dans le cas d'un mouvement intempestif de l'oeil du patient, la lentille (25), ou le système de lentilles, s'efface; et en ce que toutes les pièces du dispositif adaptable (12), susceptibles de coulisser ou de tourner les unes par rapport aux autres, sont munies d'un revêtement (31) constitué d'Al2O3 comportant des polymères PTFE intercalés et frittés.

2. Dispositif adaptable suivant la revendication 1, caractérisé en ce que le dispositif adaptable (12) présente un adaptateur (4) vissé sur le microscope (1), en ce que l'on peut installer sur l'adaptateur (4) un dispositif d'échange rapide (13), présentant un axe de rotation auquel est fixé un levier (16), sur lequel, à la même distance que l'axe de rotation, est installé, d'un côté, le dispositif adaptable (12) et, de l'autre côté, la lentille normale (11) de l'objectif du microscope.

3. Dispositif adaptable suivant la revendication 1 ou la revendication 2, caractérisé en ce que le dispositif d'échange rapide (13) est raccordé à l'adaptateur (4) au moyen d'axes de raccordement (6).

4. Dispositif adaptable suivant l'une quelconque des revendications 1 à 3, caractérisé en ce que le dispositif adaptable (12) est constitué de deux pièces de boîtier (17, 18) se vissant l'une avec l'autre et qui sont munies d'un dispositif de sécurité (21) empêchant leur séparation intempestive.

5. Dispositif adaptable suivant la revendication 4, caractérisé en ce que le dispositif de sécurité (21) est constitué d'un doigt (20), actionné par un ressort, qui est monté dans l'une des pièces de boîtier (18) et qui s'engage dans un filetage trapézoïdal (19) réalisé sur la deuxième pièce de boîtier (17).

6. Dispositif adaptable suivant l'une quelconque des revendications 1 à 5, caractérisé en ce que la position de travail de la lentille extérieure (25) est déterminée par une butée (27) installée dans la deuxième pièce de boîtier.

7. Dispositif adaptable suivant la revendication 6, caractérisé en ce que la butée (27) est constituée par une bague.

8. Dispositif adaptable suivant l'une quelconque des revendications 1 à 6, caractérisé en ce que l'axe de rotation du dispositif d'échange rapide (13) est accouplé à un moteur d'entraînement (15).

9. Dispositif adaptable suivant l'une quelconque des revendications précédentes, caractérisé en ce que le dispositif de sécurité (21) forme en même temps l'écrou fileté.
